# EUROPÄISCHE PATENTSCHRIFT

(11) **EP 0 416 159 B1**
(45) Veröffentlichungstag und Bekanntmachung des Hinweises auf die Patenterteilung: **24.05.1995**
(21) Anmeldenummer: 89116574.8
(22) Anmeldetag: 07.09.1989
(51) Int. Cl.: A61B 17/39

(54) **Mehrteilige flächenhafte Elektrode für ein HF-Chirugie-gerät**
Adhesive electrode pad for a HF surgical apparatus
Plaque-électrode collante pour un appareil de chirurgie haute fréquence

(43) Veröffentlichungstag der Anmeldung: 13.03.1991
(73) Patentinhaber: SIEMENS AKTIENGESELLSCHAFT, 80333 München (DE)
(72) Erfinder: Hagen, Uwe, Dipl.-Ing., D-8550 Forchheim (DE); Feucht, Peter, Dipl.-Ing., D-1000 Berlin 62 (DE)

(56) Entgegenhaltungen:
- EP-A- 308 690
- EP-A- 0 262 888
- CA-A- 1 219 642

## Beschreibung

Die Erfindung bezieht sich auf eine mehrteilige flächenhafte Elektrode, insbesondere neutrale Elektrode für ein HF-Chirurgiegerät, bestehend aus einem gemeinsamen biegbaren Träger und mindestens vier Teilelektroden, die einseitig am Träger angeordnet sind, wobei wenigstens eine der vier Teilelektroden im Mittenbereich des Trägers angeordnet ist und wobei die übrigen Teilelektroden außerhalb des Mittenbereiches des Trägers angeordnet sind und wobei die außerhalb des Mittenbereiches am Träger angeordneten Teilelektroden an die im Mittenbereich angeordneten Teilelektroden elektrisch getrennt angrenzen, wobei wenigstens drei Teilelektroden im wesentlichen flächenmäßig gleich groß sind und wobei diese flächenmäßig gleich großen Teilelektroden wenigstens zwei benachbarte Teilelektroden umfassen, die mit jeweils einer gleichartigen Begrenzungslinie parallel und benachbart zu wenigstens einem Abschnitt einer Umfangslinie einer der übrigen Teilelektroden angeordnet sind.

Eine mehrteilige neutrale Elektrode dieser Art ist aus der CA-A-1 219 642 bekannt, wobei in diesem Dokument Elektroden offenbart sind, mit einem kreisförmigen Träger, auf dem um eine zentrale kreisförmige Elektrode zwei konzentrische kreisringförmige Elektroden angeordnet sind oder auf dem um eine zentrale kreisförmige Elektrode zwei oder fünf konzentrische kreisringförmige Elektroden angeordnet sind, welche wegen der Elektrodenanschlüsse auf Ringabschnitten geöffnet sind, und wobei die Elektrodenanschlüsse zu einer angeformten Lasche des Trägers geführt sind. Nach einer weiteren bekannten Ausführung sind auf einem rechteckförmigen Träger sechs gleichartige, flächenmäßig gleich große, streifenförmige Teilelektroden angeordnet.
Mehrteilige Elektroden sind ferner beispielsweise aus der EP-A-0 308 690, aus der DE-A-36 23 293 und aus der DE-A-37 18 585 bekannt. Bei den aus der EP-A-0 308 690 bekannten Elektroden haben die Teilelektroden eine Kreis- oder Kreissegmentform. In jeder Elektrode haben alle Teilelektroden stets die gleiche Form und Größe. Alle Teilelektroden sind symmetrisch zu einer senkrecht auf einen im wesentlichen kreisförmigen Träger stehenden Symmetrieachse und bezüglich der Symmetrieachse in Umfangsrichtung gegeneinander versetzt angeordnet.

Um Verbrennungen beim Patienten durch teilweises Ablösen der Elektrode vom Körper des Patienten zu vermeiden, sind z.B. in HF-Chirurgiegeräten Überwachungsschaltungen üblich. Wenn der elektronische Aufwand für die jeweilige Überwachungsschaltung, z.B. Messen von Teilströmen, gering gehalten werden soll, muß eine Teilelektrode an der mehrteiligen Elektrode als Hilfselektrode zur Einspeisung eines Hilfsstromes benutzt werden. Derartige Messungen basieren auf der Überlegung, daß gleich große Teilelektroden bei vollflächigem Anliegen auf der Haut des Patienten beim Stromfluß im wesentlichen gleich große Teilströme bilden. Es handelt sich demnach um eine Symmetriemessung. Erfahrungsgemäß erfolgt eine unerwünschte Ablösung der Elektrode vom Körper des Patienten regelmäßig entlang einer geradlinig verlaufenden Ablösekante. Eine derartige Ablösekante kann im Einzelfall die aus dem Stand der Technik bekannten und verbleibenden beiden Teilelektroden so schneiden, daß beide noch nicht abgelösten Flächen der Teilelektroden jeweils eine gleich große Oberfläche haben. Wegen der dadurch entstehenden meßtechnischen Symmetrie ist das Ablösen in einem derartigen Fall mit einfachen elektronischen Schaltungen nicht erkennbar. Hier sucht die Erfindung Abhilfe zu schaffen.

Der Erfindung liegt somit die Aufgabe zugrunde, eine mehrteilige Elektrode der eingangs genannten Art so auszubilden, daß bei Verwendung einer Teilelektrode als Hilfselektrode für eine Symmetriemesssung von Teilströmen an den übrigen Teilelektroden keine Kontaktflächengleichheit bei geradliniger Ablösekante in beliebiger Richtung eintreten kann.

Diese Aufgabe wird erfindungsgemäß durch die im Patentanspruch angegebenen Merkmale gelöst. Die erfindungsgemäße Elektrode weist mindestens vier Teilelektroden auf. Wenigstens drei Teilelektroden sind flächenmäßig im wesentlichen gleich groß ausgebildet. Mit anderen Worten: Der Flächeninhalt von mindestens drei Teilelektroden ist wenigstens annähernd gleich. Wenigstens eine Teilelektrode ist im Mittenbereich des Trägers und wenigstens eine andere Teilelektrode ist außerhalb des Mittenbereiches angeordnet. An alle im Mittenbereich des Trägers angeordneten Teilelektroden grenzen alle übrigen außerhalb des Mittenbereiches angeordneten Teilelektroden elektrisch getrennt an.

Durch die erfindungsgemäße Ausbildung der mehrteiligen Elektrode kann selbst bei Auswahl einer beliebigen Teilelektrode als Hilfselektrode an den übrigen Teilelektroden keine Kontaktflächengleichheit bei geradlinig in beliebiger Richtung verlaufender Ablösekante eintreten, vor allem wegen der besonderen und erfindungsgemäßen Zuordnung der mindestens vier Teilelektroden zueinander.

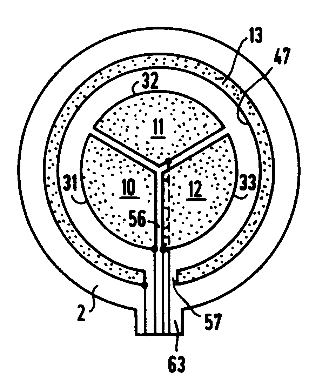

Weitere Vorteile und Einzelheiten der Erfindung ergeben sich aus der nachfolgenden Beschreibung eines Ausführungsbeispiels anhand der Zeichnung.

Die dargestellte und auf einem runden Träger 2 angeordnete erfindungsgemäße mehrteilige Elektrode besteht aus drei flächenmäßig gleich großen Teilelektroden 10 bis 12, die im Mittenbereich des Trägers 2 als elektrisch getrennte Segmente einer kreisförmigen Fläche geformt sind. Diese geometrische Fläche wird von einer kreisringförmigen Teilelektrode 13 im wesentlichen konzentrisch umschlossen. Entlang einer nicht dargestellten Umfangslinie der von den Teilelektroden 10 bis 12 bedeckten Kreisfläche sind die gleichartigen und in diesem Ausführungsbeispiel kreisbogenförmig gekrümmten äußeren Abschnitte 31 bis 33 der Begrenzungslinien der drei Teilelektroden 10 bis 12 parallel und benachbart zur inneren Umfangslinie 47 der abweichend geformten Teilelektrode 13 angeordnet. Zweckmäßigerweise wird die Teilelektrode 13, die flächenmäßig nicht gleich groß sein muß wie alle übrigen Teilektroden 10 bis 12, als Hilfselektrode benutzt, wodurch sich ein besonders symmetrischer Stromfluß über das Körpergewebe des Patienten zu den inneren Teilelektroden 10 bis 12 ergibt. Die Ringelektrode 13 weist eine Trennstelle 57 auf, zu der eine Trennstelle 56 zwischen den beiden inneren Teilelektroden 10 und 12 benachbart angeordnet ist. Dadurch ist der elektrische Anschluß über eine Lasche 63 zu allen Teilelektroden einfach realisierbar.

Die Elektrode läßt sich raum- und platzsparend herstellen und am Patienten ohne besondere Vorkehrungen auch so anordnen, daß eine weitgehend gleichmäßige Verteilung der vom Operationsfeld ausgehenden HF-Energie auf die Teilelektroden erzielt wird. Dazu trägt bei, weil alle Teilelektroden im wesentlichen symmetrisch zu einer imaginären Achse angeordnet sind, die im Mittenbereich des Trägers 2 zur gemeinsamen Ebene der Teilelektroden senkrecht steht. Eine Flächengleichheit der jeweils benachbarten Teilelektroden 10 bis 12 läßt sich besonders einfach erreichen, wenn diese Teilelektroden jeweils eine durch geradlinige Begrenzungskanten gebildete Elektrodentrennstelle 56 aufweisen, deren jeweilige Längsrichtung senkrecht zu dem jeweils benachbarten Abschnitt der inneren Umfangslinie 47 der kreisringförmigen Teilelektrode 13 steht. Nach der Erfindung ausgebildete mehrteilige Elektroden lassen sich auch in Verbindung mit aufwendigeren und weiteren elektronischen Überwachungsschaltungen, z.B. für Impedanzmessungen, verwenden, wodurch auch Kompatibilität zu unterschiedlichen Überwachungsschaltungen an HF-Chirurgiegeräten erreichbar ist.

## Patentansprüche

1. Mehrteilige flächenhafte Elektrode, insbesondere neutrale Elektrode für ein HF-Chirurgiegerät, bestehend aus einem gemeinsamen biegbaren Träger (2) und mindestens vier Teilelektroden (10 bis 13), die einseitig am Träger angeordnet sind, wobei wenigstens eine der vier Teilelektroden (10 bis 12) im Mittenbereich des Trägers angeordnet ist und wobei die übrigen Teilelektroden (13) außerhalb des Mittenbereiches des Trägers angeordnet sind und wobei die außerhalb des Mittenbereiches am Träger angeordneten Teilelektroden an die im Mittenbereich angeordneten Teilelektroden elektrisch getrennt angrenzen, wobei wenigstens drei Teilelektroden (10 bis 12) im wesentlichen flächenmäßig gleich groß sind und wobei diese flächenmäßig gleich großen Teilelektroden wenigstens zwei benachbarte Teilelektroden (10 bis 12) umfassen, die mit jeweils einer gleichartigen Begrenzungslinie (31 bis 33) parallel und benachbart zu wenigstens einem Abschnitt einer Umfangslinie (47) einer der übrigen Teilelektroden (13) angeordnet sind, **dadurch gekennzeichnet,** daß eine kreisringförmige Teilelektrode (13) eine kreisförmige Fläche im wesentlichen konzentrisch umschließt, die im Mittenbereich des Trägers (2) drei voneinander getrennte Kreissegmente aufweist, die drei benachbarte sektorförmige Teilelektroden (10 bis 12) bilden.

## Claims

1. Multi-component planar electrode, in particular a neutral electrode for an HF surgical instrument, consisting of a common flexible carrier (2) and at least four component electrodes (10 to 13) which are arranged on one side of the carrier, whereby at least one of the four component electrodes (10 to 13) is arranged in the central region of the carrier and whereby the remaining component electrodes (13) are arranged outside the central region of the carrier and whereby the component electrodes arranged on the carrier outside the central region adjoin in electrically separated manner the component electrodes arranged in the central region, whereby at least three component electrodes (10 to 12) are substantially of equal size in terms of surface area and whereby these component electrodes of equal surface area include at least two adjacent component electrodes (10 to 12) which are arranged with, in each case, a similar boundary line (31 to 33) parallel to and adjoining at least one section of a peripheral line (47) of one of the remaining component electrodes (13), characterised in that an annular component electrode (13) surrounds in substantially concentric manner a circular surface area exhibiting in the central region of the carrier (2) three circular segments which are separated from one another and formed by three adjacent sector-shaped component electrodes (10 to 12).

## Revendications

1. Électrode de surface étendue formée de plusieurs éléments, notamment électrode neutre pour un appareil chirurgical à haute fréquence, constituée par un support commun flexible (2) et par au moins quatre électrodes partielles (10 à 13), qui sont disposées d'un côté sur le support, et dans laquelle au moins l'une des quatre électrodes partielles (10 à 13) est disposée dans la partie centrale du support, les autres électrodes partielles (13) étant disposées à l'extérieur de la zone centrale du support, que les électrodes partielles, disposées à l'extérieur de la zone centrale du support, jouxtent, tout en en étant séparées électriquement, les électrodes partielles disposées dans la zone centrale, au moins trois électrodes partielles (10 à 12) possèdent des surfaces essentiellement identiques et ces électrodes partielles possédant des surfaces essentiellement identiques comprennent au moins deux électrodes partielles voisines (10 à 12), qui sont disposées de telle sorte que respectivement une ligne limite identique (31 à 33) s'étend parallèlement et au voisinage d'une partie d'une ligne périphérique (47) de l'une des autres électrodes partielles (13), caractérisée par le fait que l'électrode partielle en forme d'anneau circulaire (13) entoure essentiellement concentriquement une surface circulaire qui possède, dans la zone centrale du support (2), trois segments circulaires séparés les uns des autres, qui forment trois électrodes partielles voisines en forme de secteurs (10 à 12).
